# EUROPEAN PATENT APPLICATION

(11) **EP 3 763 287 A1**
(43) Date of publication of application: **13.01.2021**
(21) Application number: 19185270.6
(22) Date of filing: 09.07.2019
(51) Int. Cl.: A61B 5/055, G01R 33/54, A61B 5/00, G16H 40/63

(54) **PATIENT CONTROLLED MEDICAL SYSTEM**

(71) Applicant: Koninklijke Philips N.V., 5656 AG Eindhoven (NL)
(72) Inventor: LEUSSLER, Christoph, 5656 AE Eindhoven (NL); AMTHOR, Thomas Erik, 5656 AE Eindhoven (NL); MAZURKEWITZ, Peter, 5656 AE Eindhoven (NL)
(74) Representative: de Haan, Poul Erik

(57) **Abstract**

A medical system (300) comprises a medical examination or treatment apparatus (302) and a wearable patient device (100). The medical examination or treatment apparatus (302) comprises an examination or treatment zone for a patient (304), and the wearable patient device (100) comprises a user interface operable by a hand of the patient when the patient (304) is positioned in the examination or treatment zone of the medical examination or treatment apparatus (302). The wearable patient device (100) is communicatively connected with the medical examination or treatment apparatus (302) via a wireless connection, for sending a control command corresponding to input received from the patient via the user interface of the wearable patient device (100), the control command being adapted to control a patient-controllable part or parameter (308) of the medical examination or treatment apparatus (302).

## Description

### FIELD OF THE INVENTION

The invention relates to a wearable patient device of a medical examination or treatment system, a corresponding medical examination or treatment system, a corresponding computer program product, and a method of patient-control of the medical system.

### BACKGROUND OF THE INVENTION

Various medical imaging modalities such as computer tomography (CT), magnetic resonance imaging (MRI), positron emission tomography (PET), and others can be used for imaging the internal anatomy of a subject. For example, a strong static magnetic field is used by Magnetic Resonance Imaging (MRI) scanners to align the nuclear spins of atoms as part of the procedure for producing images within the body of a subject. Various quantities or properties of the subject can be measured spatially using MRI. However, the acquisition of a magnetic resonance imaging data and other types of medical imaging data is not instantaneous. The subject may need to remain as motionless as possible for a number of minutes.

More importantly, many medical imaging or treatment devices are located in rooms that are heavily shielded against electromagnetic and other radiation. Such shielding almost invariably also provides acoustic shielding.

Thus, while undergoing a medical procedure involving a medical examination or treatment device in a room that is acoustically as well as electromagnetically and/or radiation shielded from a room in which an operator or caregiver is present it may be difficult for patients to communicate with the operator or caregiver. Communication between operator or caregiver and patient may be desirable to instruct the patient to perform particular actions or movements or the like, or to inform the patient about a current status and progress of an examination or treatment.

Some medical procedures take rather long time periods. There may thus be a need for the patient to change properties of a part of a medical examination or treatment device used during the medical procedure that receives the patient during the procedure, e.g., a patient table, patient support, or a cushion thereof, or change properties of a room in which the medical procedure is performed, to improve comfort and well-being during the medical procedure. Communication between the patient and the operator may thus be desirable to request the operator to change such environmental settings of the shielded room or the medical instrument. Communication between the patient and the operator may further be desirable simply for enabling the patient to notify the operator about a bodily status or general wellbeing.

Some medical imaging devices are equipped with video displays for informing or instructing patients, for providing visual stimuli during the imaging procedure and the like. Some medical imaging devices are equipped with microphones and headphones for the patient, providing a means of bidirectional voice communication between the patient and an operator. However, some types of medical examinations or treatments do not permit carrying a headphone, and typically leaving the microphone activated during the entire scan procedure is undesirable, because all kinds of noises generated by the medical imaging device during the scan will be picked up and may distract the operator. Also, a patient's voice may be low or muffled, which would require amplifying the signals picked up by the microphone, including the machine noise.

Nurse call systems are usually provided with medical imaging or treatment devices for informing an operator about issues of discomfort, who can then activate a voice communication channel to the patient. One known nurse call system uses an air-filled compressible member, e.g. a small balloon-like air ball, that the patient holds in his or her hand. The compressible member is connected to a hose or tube that has a pressure sensor for detecting when the patient compresses the compressible member. When a change in the pressure in the hose or tube is detected, a signal is output that informs the operator about the need to communicate with the patient. Simpler versions may use a whistle located in a control room that is connected to the hose or pipe and which produces an acoustic signal when the patient compresses the compressible member. The operator can then activate the voice communication in response to the signal.

While the known system provides a simple means of generating an unspecific alert signal, the hose or tube may be clamped or pinched between moving parts of the medical imaging device, effectively cutting off the pressure sensor or the whistle from the compressible member and thus interrupting the only way the patient can initiate a communication with the operator. Further, the compressible member must be held in the patient's hand at all times during the treatment or examination, which may be challenging for some patients, notably patients with motoric or other disabilities. If the compressible member is lost, the only possibility for the patient to initiate a communication is lost. In addition, patients that are hearing impaired, speech impaired and/or visually impaired may not be able to make proper use of the video display or the voice interface. Yet further, the known system can only provide a single signal and is thus limited in its application.

It is, therefore, desirable to provide improved means for patient communication with operators of medical devices, medical devices and/or systems, and for patient control of parameters or settings of a medical device and/or system.

### SUMMARY OF THE INVENTION

The invention provides for a medical system comprising a medical examination or treatment apparatus and a wearable patient device communicatively connected thereto. The invention further provides for a method of controlling the medical examination or treatment device using the wearable patient device, and a computer program product in the independent claims. Embodiments may provide a means for a patient to regulate or control patient-modifiable parameters of the medical examination or treatment system. Further embodiments and developments are provided in the dependent claims.

The disclosed medical system comprising a medical examination or treatment apparatus and a wearable patient device may enable a patient for wirelessly sending a control command to the medical examination or treatment apparatus and optionally also to wirelessly communicate with an operator of the medical examination or treatment apparatus. Thus, instead of having e.g. a nurse call system that uses the above-described air-filled compressible balloon, the patient may be able to communicate more comfortably and more flexibly by means of the wearable patient device. Furthermore, he will gain limited "control" over the examination procedure carried out using the medical examination or treatment apparatus.

In one aspect the present invention provides for a medical system comprising a medical examination or treatment apparatus and a wearable patient device communicatively connected thereto. The medical examination or treatment apparatus has an examination or treatment zone for a patient. The wearable patient device comprises a user interface operable by a hand of the patient when the patient is positioned in the examination or treatment zone of the medical examination or treatment apparatus. The wearable patient device is further communicatively connected with the medical examination or treatment apparatus via a wireless connection, for sending a control command corresponding to input received from the patient via the user interface of the wearable patient device. The control command is adapted to control a patient-controllable part or parameter of the medical examination or treatment apparatus.

In the context of the present disclosure a "medical examination or treatment apparatus" may relate to an apparatus temporarily engaged with the patient. The engagement may include bodily contact between the patient and at least a part of the medical examination or treatment device, e.g. a sensor collecting patient data for diagnosis, an actuator or emitter used for treating the patient, or a patient support in or on which the patient is located during the diagnosis or treatment, but may also include an engagement for treatment or diagnosis that does not require bodily contact, e.g. any kind of contactless imaging or radiation treatment or the like.

The controlling of the patient-controllable part may comprise one or more of interrupting or aborting an examination or treatment, controlling at least a temperature or a softness of a cushion of a patient bed or patient support for positioning the patient in the examination or treatment zone, and of a system for illuminating, ventilating or odorizing of the examination or treatment zone. The controlling of the patient-controllable part may also comprise controlling systems associated with the medical examination or treatment device. Such system may comprise a room or location in which the medical examination or treatment apparatus is placed, as well as sub-systems that control environmental or other properties of the room or location, e.g., illumination, temperature, ventilation, acoustics, and the like. While other forms of association, physical or logical, are not excluded herein, association may comprise association by location. Further, entertainment systems for use by the patient during the treatment or examination maybe considered systems associated with the medical examination or treatment apparatus.

In at least one embodiment patient input may comprise operation of buttons or switches of the input means by the patient, but also input generated without explicit control through the patient, e.g., by sensors, switches or contacts comprised in the wearable patient device that are configured to monitor physiological or other parameters of the patient, or that implement means for monitoring statuses of the wearable patient device.

Input means in one or more embodiments may thus include mechanical switches, electric switches or electronic switches, e.g., optical or capacitive switches, that provide binary signals, or include conducting polymers that may produce a variable continuous or multi-level output signal depending on applied pressure or deformation.

In one or more embodiments the input means of the wearable patient device may further comprise one or more sensors configured to detect physiological properties or parameters of the patient. The wearable patient device may be adapted for transmitting via the wireless connection a signal representing the sensor input, or derivatives thereof, to the medical examination or treatment apparatus, and the medical examination or treatment apparatus may be adapted to modify its operation in response to the received signal.

In one or more embodiments the sensors of the wearable patient device may comprise sensors for determining as the physiological properties or parameters a patient's heart rate or pulse, blood pressure, blood oxygenation, blood glucose level, skin conductivity, temperature, and/or may comprise gyroscopes or accelerometers. Other input means may include fibre-optical shape sensing means, pressure sensors, as well as various other types of sensors for determining physiological and other parameters of the patient, including patient's motion or movements, like for example accelerometers or gyroscopes.

The input means of one or more embodiments may also be configured to permit gesture recognition, e.g. by detection of movements and/or positions of the hand, fingers and/or phalanges.

Patient input representing statuses of the wearable patient device may include generating signals representing one or more operating states, a state of attachment of the wearable patient device to the patient, a state of an internal power supply, a state after self-testing, and the like. The state of attachment of the wearable patient device to the patient may indicate when the wearable patient device is about to become loose from the patient, and may be used for triggering an alarm or aborting a treatment or examination. The state of attachment maybe determined, e.g., by using corresponding electrical or optical switches, by analyzing signals from sensors detecting physiological parameters of the patient, or by analyzing signals from a motion detector to detect motion patterns indicating, e.g., a loose fit of the wearable patient device or the like.

In one or more embodiments a self-test of the wearable patient device may be initiated upon attaching the wearable patient device to the patient and/or may be cyclically repeated, including performing self-tests in a fashion that is timing-synchronized with one or more states or phases of an examination or treatment. Generally, patient input may include any signal that is generated through or by the wearable patient device and that is transmitted to a remote receiver.

Modifying the operation of the medical examination or treatment apparatus may comprise one or more of interrupting or aborting an examination or treatment of the patient by the medical examination or treatment apparatus, or triggering an examination or treatment or a particular variant thereof of the patient by the medical examination or treatment apparatus. This may be useful, e.g., for causing an examination device to perform or stop a medical scan upon the patient inputting a command to the wearable patient device or upon the sensor data indicating presence of a predetermined physiological condition of the patient. For example, it maybe possible to trigger or gate a scan whenever a physiological parameter, a pulse rate or a blood oxygenation, is in a predetermined suitable range, or to cancel a scan and purge scan data when the physiological parameter is outside a predetermined suitable range. The information about the conditions upon which the scan has been triggered may be used in a later processing stage, e.g., may be provided as additional input to an artificial intelligence that processes the scan data.

The wearable patient device may further be communicatively connected with a communication system providing voice communication between the patient and an operator of the medical examination or treatment device, and the wearable patient device may be configured to establish or initiate the voice communication. Voice communication may be established between the patient and an operator of the medical examination or treatment device. An operator of the medical examination or treatment device may include, inter alia, a caregiver monitoring the medical examination or treatment device and/or the patient during the examination or treatment, or an operator controlling the operation of the medical examination or treatment device.

The wearable patient device includes a communication module that is configured to transmit communication and/or control signals to at least one receiver and to receive communication and/or control signals from at least one transmitter, the receiver and/or transmitter being in signal connection with the medical examination or treatment apparatus or systems associated therewith.

In at least one embodiment the communication module may be configured for wireless or wired communication. Wireless communication may be based on radiofrequency transmission in accordance with commonly known standards, as long as the operating frequency is sufficiently far away from or outside of a frequency range used by the medical examination or treatment device. Alternatively, optical transmission, e.g. via accordingly modulated infrared or visible light, or acoustical transmission, e.g. using modulated ultrasonic signals may be used for wireless communication. Wired transmission may use shielded electrically conducting wires, or optical transmission using light guides or fibre-optic lines. Other known and suitable wireless and wired transmission means may be used.

Sensor input, or signals derived therefrom, maybe transmitted, via the communication module of the wearable patient device and the receiver and/or transmitter of the system, to the medical examination or treatment device or a patient-controllable part thereof, to systems associated with the medical examination or treatment device, and/or to a data storage or data processing system, for causing changes of the operation during the medical examination or treatment in response to the sensor signals and/or for later analysis. Causing changes of the operation may include causing the operation to be aborted or terminated, but also adaptation of the operation in response to the sensor signals.

In addition to input means for receiving patient input the user interface of the wearable patient device may comprise output means for providing feedback to the patient.

In at least one embodiment the output means may include means providing haptic, acoustic and/or visual information and/or feedback. Haptic information or feedback may be provided through a vibrator or knocker, or through mild electric stimulation of skin nerves. Acoustic information or feedback may for example be provided through a buzzer or a loudspeaker. Acoustic feedback may be enabled or disabled in dependence of an operating state of the medical device, e.g. may be disabled when the medical examination or treatment device produces noise and enabled at other times when the medical examination or treatment device produces little or no noise. Visual information feedback may be provided through a modulated light source, e.g., a flashing or pulsing light emitting diode (LED). Such visual feedback may preferably be used in situations when the ambient light is low.

In the context of the present disclosure the term "feedback" may refer to a confirmation provided in response to a patient input received via the patient interface, but also to a confirmation provided from a caregiver or operator of the medical device. The term "information" may refer to an instruction provided by a caregiver or operator of the medical examination or treatment device without prior input or request by the patient, e.g., for signaling a patient to perform a specific action or task, or for guiding the patient during the performance of such specific action or task.

Feedback or information provided to the patient, in particular optical or haptic feedback or information, may be coded, e.g. as a pulse sequence, a pulse or vibration pattern involving pulses and pauses in between, by modulation of the amplitude, frequency and/or length of a pulse, etc. Such feedback or information may be used for providing guidance to the patient, e.g., when to inhale, when to exhale, and when to hold the breath. Such guidance may for example be of great value for diagnostic imaging in the thorax region, for reducing morphologic distortion and diagnostic failure / misinterpretation (e.g. hypertrophy in cardiac MRI) or to ensure accurate lesion localization.

Each patient action may be indicated and guided by a specific haptic or optic information or feedback pattern that is designed for easy identification and following by the patient, eliminating the need to have a voice communication between the patient and the operator. Becoming familiar with patient actions as well as with providing patient input for controlling the medical examination or treatment device or systems associated therewith may be subject to a patient training, as will be discussed further below in this document.

As monotonous noises that may be audible during an examination or treatment may lead to patients falling asleep, which can strongly deteriorate compliance, the feedback or information provided through the patient interface may also be used for keeping patients awake.

In one or more embodiments at least the patient user interface of the wearable patient device is provided in an enclosure that is removably attachable to a lower part of a patient's arm, e.g. a hand, such that the patient can operate the patient interface using the hand of that arm. Removably attachable may refer to an attachment that does not require the patient to actively hold the enclosure or the wearable patient device in a hand. Holding objects in a hand for extended periods of time may cause cramps and may have an impact on the responsiveness of the hand when feedback from the patient is requested. Exemplary enclosures may include sleeve-like, cuff-like, wrist band-like or glove-like enclosures. The enclosures may be configured to be opened or separated for inserting a part of the patient's arm, and/or may have a flexible portion that allows inserting a part of the patient's arm without opening or separating the enclosure.

The patient user interface is preferably configured to permit operation of the input means using the hand and/or the fingers of the hand of the arm to which the patient interface is attached. Such configuration eliminates the need for a patient to move both arms for operating the input means, reducing the movement required for operating the input means to a minimum. Reduced movement of patients is of particular importance during diagnostic imaging or therapeutic treatments that need to maintain focus on small areas for an extended period of time, and can thus reduce the effort for tracking a patient's position or posture.

In other embodiments the patient user interface is removably attached to a part of the patient's body that can be reached by a minimum movement of the hand of the patient while positioned in an examination or treatment apparatus, e.g., at a thigh of a patient.

In one or more embodiments the patient interface is configured to receive, via the input means, a request to initiate or establish a communication between the patient and a caregiver or operator of the medical examination or treatment device. The request may be received from the patient, e.g., in response to the patient operating a corresponding switch or control button of the patient interface, or as a result of analysis of one or more sensor signals, e.g., in case of untypical sudden motion or absence of motion, in case of suddenly increased or decreased pulse rate, temperature, skin conductivity or the like, and combinations thereof. The latter exemplary case may implement an automatic nurse call or emergency call. A microprocessor or computer that is provided in the wearable patient device may execute corresponding computer program instructions stored in an associated memory of the wearable patient device, which implement a corresponding signal analysis module.

In one or more embodiments the patient interface is configured to receive, via the input means, patient input for controlling patient-controllable environmental parameters of systems associated with the medical examination or treatment device and/or for controlling patient-controllable parameters or parts of the medical apparatus. Input for controlling patient-controllable parameters of systems associated with the medical examination or treatment device may be targeted to adjust or select illumination, temperature, ventilation and/or odorization of a room in which the patient and the medical examination or treatment device is located. Exemplary input for controlling patient-controllable parameters of the medical examination or treatment device may be targeted to adjust or select entertainment options, e.g., music volume or selection of music styles etc., illumination or odorization of an examination chamber or space of the medical examination or treatment device in which the patient is received, temperature or softness of a cushion of a patient support, and the like. Patient input may also be used for changing parameters of the medical device, e.g. sequence parameters in an MRI scanner in case the peripheral nerve stimulation (PNS) experienced by the patient is too high. Adjustments to patient-controllable parameters maybe limited in selection and/or range, either generally for all patients or individually for each patient. Corresponding limits may be stored in the wearable patient device, in each patient-controllable system, or in a central system controller that may be provided in some embodiments.

In one or more embodiments the patient interface of the wearable patient device is configured to receive patient input to other stimuli provided or presented to the patient, and to transmit the received input to a system that analyzes parameters of the patient input, e.g., timeliness, correct type of patient input, etc., in relation to the respective stimulus. The analyzing system may be communicatively connected to the wearable patient device via the communication module and the receiver and/or transmitter. A so-configured patient interface may be useful in medical experiments in connection with a treatment or examination.

In one or more embodiments the wearable patient device is configured to provide an interface for connecting a voice communication means. Such interface may include a connector for connecting a headphone or headset, either electrically or acoustically. A headset or headphone of the latter type may comprise tubes made from a non-magnetic material that are conducting acoustical waves from a transducer located in the wearable patient device. Locating an acoustic transducer in the wearable patient device may facilitate shielding against interferences. The voice communication signals may be transmitted from the communication module of the wearable patient device via the at least one receiver and/or transmitter to a communication system near an operator of the medical examination or treatment system. The voice communication means may also serve for patient entertainment, e.g., for listening to music or the like. In this case signals from an entertainment source that represents a system associated with the medical examination or treatment device are transmitted to the wearable patient device via the at least one receiver and/or transmitter.

In one or more embodiments the communication module of the wearable patient device is configured for temporary coupling or connecting to a medical examination or treatment apparatus. Coupling may include a logical coupling or connecting to logically implemented receivers and/or transmitters via a shared physical interface, but also establishing and maintaining multiple separate physical connections.

Temporary coupling or connecting may be useful in case the wearable patient device is attached to the patient while another patient is being examined or treated, and the other patient's wearable patient device is coupled to the medical examination or treatment apparatus. In this case a wearable patient device may receive coupling information or enable coupling only briefly before the treatment can be started, and after a previously conducted examination or treatment has ended. In some embodiments the coupling information is provided or coupling is enabled only after a patient has successfully completed a training involving the wearable patient device.

In one or more embodiments the wearable patient device is configured for coupling to a single physical receiver and establishing and maintaining respective logical connections to the medical examination or treatment apparatus, systems associated with the medical examination or treatment apparatus and/or for initiating a communication channel between the patient and the operator. This may be useful in case a central system controller is provided, which is in communicative connection with the medical examination or treatment apparatus, systems associated with the medical examination or treatment apparatus and a communication system provided for communication between the patient and the operator. The single physical receiver may be coupled to the system controller and may receive control and communication signals targeted to any of the systems or devices it is connected to, and forward or route the signals to the appropriate device or system. A single physical connection may also facilitate provision and integration of a simulator for training patients.

The wearable patient device may have a microprocessor, associated volatile and non-volatile memory storing computer program instructions for controlling the communication module and the patient interface.

The wearable patient device may have an internal power source, e.g. a battery, preferably a rechargeable battery or a battery that can be easily replaced. A connector for recharging the battery can be provided, preferably covered by a removable cover, for facilitating cleaning and disinfection of the wearable patient device. Alternatively, a circuit for wireless charging may be provided in the wearable patient device, which does not require any opening in the enclosure and thus provides for easy cleaning and disinfection. Termination of the charging may produce a signal that may be used for initiating a change in the operation mode of the wearable patient device, e.g., entering association or coupling mode, or the like. The charging status of the device may be indicated optically, e.g., numerically as a percentage, or simply through a linear arrangement of two or more LEDs that indicate a fully or half charged battery, or an empty battery. The LEDs may also provide a blinking or flashing pattern during charging and trickle charging, the latter also indicating a completed charging process.

According to another aspect a system is provided having a medical diagnostic or treatment apparatus placed in an examination or treatment room that may be shielded against magnetic fields, electro-magnetic and/or other radiation and/or acoustically, and that further comprises at least one receiver and/or transmitter located inside the examination or treatment room. The at least one receiver and/or transmitter is configured to communicatively connect with a wearable patient device as presented hereinbefore. The at least one receiver and/or transmitter may be configured for providing communication to the outside of the examination or treatment room.

The receiver and/or transmitter for providing communication to the outside of the examination or treatment room have a first communication channel to the outside of the examination or treatment room. The first communication channel at least provides a connection between a wearable patient device as presented hereinbefore that is located inside the examination or treatment room and connected to the receiver and transmitter and a communication device in a control room that is separate from the examination or treatment room. The communication device in the control room at least provides one or more of an acoustic, a visual and a haptic signal to an operator or caregiver who monitors or controls the examination or treatment. A device or system for unidirectional or bidirectional voice communication between the control room and the treatment or examination room may be provided. The voice communication system may communicate via the first communication channel or via a separate communication channel.

In embodiments the system may comprise further receivers and transmitters located in the examination or treatment room, which are connected to or part of the medical examination or treatment apparatus or systems associated with the medical device. The further receivers and transmitters may establish and maintain individual communication connections, physical or logical, with embodiments of wearable patient devices as presented hereinbefore.

In embodiments the connection between the wearable patient device in the examination or treatment room and the communication device in the control room may be established via a central system controller that is connected to the at least one receiver and/or transmitter in the shielded room. The central system controller may further be communicatively connected with the medical examination or treatment device or the patient-controllable part thereof, one or more systems associated with the medical device, and/or a hospital information system, which may have communication modules configured to receive communication and/or control signals from the wearable patient device via or under control of the system controller, or to transmit communication and/or control signals to the wearable patient device via or under control of the system controller. The wearable patient device may accordingly transmit signals for controlling patient-controllable elements or functions of the medical device, the systems associated with the medical examination or treatment device or for establishing communication with an operator or caregiver to those systems via or under control of the system controller, which establishes a connection with or which routes the signals to the appropriate receiver. Signals from the medical device, the systems associated with the medical device, the communication system of the operator or caregiver and/or the hospital information system are correspondingly routed via the system controller or under control thereof to the wearable patient device or other appropriate receivers or via corresponding communication channels established or controlled by the system controller. Establishing or controlling communication channels may comprise providing channel information, authorization and/or authentication data to the systems that are party to the respective communication.

The central system controller may be configured to establish a temporary logical association between a wearable patient device as discussed hereinbefore, a medical examination or treatment device and/or one or more systems associated with the medical device. The central system controller may also use patient data from the hospital information system for such temporary assignment. The wearable patient device, the patient, the medical examination or treatment device and systems associated with the medical examination or treatment device may have unique identifiers that may be used for the logical association. These unique identifiers may also be part of messages transmitted by any of the devices, in particular in messages carrying sensor data or the like that is received, recorded and/or stored. Identification means attached to one or more of the components and elements of the system and carrying the unique identifiers may include near field communication (NFC) tags, radio frequency identification (RFID) tags, machine readable graphical codes, e.g., bar codes or QR-codes, tags carrying text information suitable for optical character recognition, or the like. The patient identification may also involve a biometric identification, e.g. a fingerprint or a face recognition, that is verified against corresponding data stored in a database, e.g., the hospital information system.

Appropriate readers for the identification means may be communicatively connected to the central system controller and activated at least in a set-up and association phase. This may comprise readers that are provided with the components that are to be temporarily logically associated. For example, a wearable patient device as presented herein may have a reader for an identification means, e.g., a feature or tag that carries a patient ID, and transmit the read identifier to the central system controller. However, it is likewise possible to provide a separate reader communicatively connected or connectable to the central system controller that is configured to read identification means of all components or elements that are to be temporarily logically coupled and that transmits them to the central system controller or the individual components or elements, for creating the temporary association.

If no central system controller is involved in the coupling, the wearable patient device, the receiver and/or transmitter of the communication system, corresponding receivers and/or transmitters of the medical examination or treatment device and of systems associated with the medical examination or treatment device may be coupled by reading the identifiers from the identification means discussed hereinbefore using appropriate means configured for reading the identification means.

In according embodiments, the wearable patient device has a reader for reading the identification means of the patient and other components required for a training or an examination or treatment. The identifier of each component may specify a communication channel, physical or logical, that can be used for communicating with the respective component. In these embodiments the wearable patient device may be central for setting up the system.

Unique identifiers allow for preparing a second patient for an impending examination or treatment while examination or treatment of a first patient is still ongoing, thereby improving the hospital workflow. This includes the second patient performing training while the first patient is examined or treated in a nearby examination or treatment room without having to rely on the shielding properties of the treatment room, since the logical association is based on the unique identifiers of the components. Any undesired interaction between two patients and the systems for examination or treatment and the training system can be avoided through the use of the unique identifiers and the logical association of elements by their identifiers.

The logical association between the various components of the system may be initiated upon one or each of the components to be associated receiving an according input. The input may be a user input, e.g. operation of a switch or control, or may be a signal received from one of the components, or a signal generated when handling a component. A signal of the latter kind may for example be generated when a battery-powered component is removed or disconnected from a battery charger, or when a sensor provided for detecting physiological parameters of the patient detects such parameters for the first time after a previous deactivation or disconnection. The association may need to be completed within a predetermined time period after initiation.

In some embodiments, in order to associate a patient, a wearable patient device as presented herein and components or elements of the examination or treatment system the identification means of the various components or elements are read by suitable readers or scanners, and the identifiers of the temporarily associated components or elements are notified to a system controller that manages the communication and/or to the individual associated components or elements. The temporary association is registered and stored for later use or reference, e.g., in a hospital information system. Each of the associated components or elements may add its own identifier to any communication message transmitted in order to ensure that messages and information carried therein can be associated with the proper patient or device.

According to yet another aspect, in some embodiments a preparation room is provided, comprising a training system or system simulator. The training system or system simulator has a receiver and transmitter for communicatively coupling to a wearable patient device as discussed hereinbefore, e.g. using the identification means previously discussed. The training system or system simulator may comprise a computer that is configured to simulate phases of an impending examination or treatment of the patient as well as patient interactions that may occur during those phases of the examination or treatment. The training system or system simulator may also comprise means for providing visual or acoustic instructions or feedback to the patient during the training, e.g., one or more displays or loudspeakers.

Simulating the impending examination or treatment may comprise receiving patient input relating to patient-controllable parameters of the medical examination or treatment device or systems associated with the medical examination or treatment device at any time, and providing a response to the patient. The response may be provided by controlling output means of the wearable patient device or via a separate display and/or loudspeaker or buzzer.

Simulating the impending examination or treatment may also comprise transmitting instructions to the patient, and receiving signals from the wearable patient device in response. Instructions to the patient may include, inter alia, instructions for inhaling, exhaling and holding the breath. Such instructions may be provided by accordingly operating a haptic feedback element of the patient interface of the wearable patient device, e.g., a vibrator. Signals received in response to instructions may include, inter alia, signals from sensors or from switches or buttons operated by the patient.

Training the patient through a simulation of the impending treatment or examination and the communication and control functions may help increasing the patient's confidence in the technology and ensuring a smooth course of the examination or treatment. The success of the training may be determined by analyzing various aspects of the patient's responses to corresponding stimuli.

In some embodiments the actual medical treatment or examination may only be started after one or more functions of the wearable patient device have been successfully tested. In one or more embodiments a successful test of the function for initiating or establishing a communication with a caregiver or operator of a medical examination or treatment device used for examination or treatment of the patient, often also referred to as nurse call, is a mandatory requirement for starting the medical treatment or examination. Testing of functions may help identifying and replacing defective or otherwise non-functional wearable patient devices before an examination or treatment is begun, thus improving patient safety and ensuring a smooth workflow.

The medical examination or treatment device and/or the wearable patient device may accordingly comprise computer program instructions or receive corresponding signals that prevent starting the medical examination or treatment until the communication functionality of the wearable patient device has been successfully tested and/or until the patient has performed training on the wearable patient device.

In some embodiments data pertaining to the training, the logical association of devices, including which devices and patients are coupled or who performed or supervised the logical association procedure, and to patient interactions during the treatment or examination are recorded and stored for later reference. Recording may comprise recording message flows between components and capturing video images of the process. This may be used for further analysis of workflows, identifying components causing trouble, and a plethora of other purposes.

In some embodiments the patient input through the wearable patient device may be used for indicating a "patient readiness", i.e., a signal from the patient that she or he is ready for beginning the treatment or examination. This may be useful, e.g., in case a patient has a high pulse rate or is generally agitated when entering the examination or treatment room and needs time to calm down before treatment or examination can be started. Giving the patient more control over the situation may help increasing patient comfort.

In some embodiments a patient may have one wearable patient device attached to each arm, thereby increasing the options for providing feedback to the patient and/or receiving input from the patient. Also, the number of control options offered to the patient may be higher. Preferably, each of the wearable patient devices is configured to be operated using the hand of the same arm, in order to reduce the movements necessary for operation.

Each of the components or elements presented hereinbefore may comprise one or more microprocessors and associated volatile and non-volatile memory, data interfaces to internal and external components, and the like. The non-volatile memory may store machine-executable instructions at least for controlling or supporting the temporary association of the components or elements and the communication between the components or elements.

In yet another aspect the invention provides for a computer program product comprising machine-executable instructions for execution by a processor controlling at least a communication module of a wearable patient device, of a system, or of a training system as presented herein. The machine executable instructions at least configure the respective communication modules of one of the processor-controlled devices, patient-controllable parts thereof, or systems, to establish a communication connection with at least one respective other device, patient-controllable part thereof, or system, and to receive control and communication signals from and transmit such signals to the wearable patient device.

It is understood that one or more of the aforementioned embodiments of the invention may be combined as long as the combined embodiments are not mutually exclusive.

As will be appreciated by one skilled in the art, aspects of the present invention may be embodied as an apparatus, a system, method or computer program product. Accordingly, aspects or embodiments of the present invention may take the form of an entirely hardware-implemented embodiment, an entirely software-implemented embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

Aspects of the present invention are described with reference to message flow illustrations and/or block diagrams of methods, apparatus (systems) and computer program products according to embodiments of the invention. It is understood that each block or a portion of the blocks of the flowchart, illustrations, and/or block diagrams, may be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein may be an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of user input through a switches or contacts, touch-sensitive inputs and accelerometer are all examples of user interface components which enable the inputting of information or data by an operator.

A 'communication interface' as used herein encompasses an interface which enables the processor of a computer system to interact with and/or control an external computing device and/or apparatus. A communication interface may allow a processor to send control signals or instructions to an external computing device and/or apparatus. A communication interface may also enable a processor to exchange data with an external computing device and/or apparatus. Examples of a communication interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as two or three-dimensional data that has been acquired using a medical imaging scanner or a medical imaging system. A medical imaging scanner or system is defined herein as an apparatus adapted for acquiring information about the physical structure of a patient and construct sets of two dimensional or three-dimensional medical imaging data. Medical imaging data can be used to construct visualizations which are useful for diagnosis by a physician. This visualization can be performed using a computer.

Magnetic Resonance Imaging (MRI) data is an example of medical imaging data and is defined herein as being the recorded measurements of radio frequency signals emitted by atomic spins using the antenna of a Magnetic resonance apparatus during a magnetic resonance imaging scan. A Magnetic Resonance image or MR image is defined herein as being the reconstructed two or three-dimensional visualization of anatomic data contained within the magnetic resonance imaging data. This visualization can, for example, be performed using a computer.

As is apparent from the foregoing discussion the proposed wearable patient device and systems connectable to the wearable patient device may help improving the workflow in a hospital or clinic, reliability of the treatment or examination and patient comfort. Enabling a patient to reliably initiate a voice communication with an operator while inside an examination or treatment room improves the patient's well-being and feeling of safety. Enabling a patient to control some components or elements while in the examination or treatment room further increases the well-being of the patient by leaving the patient less helpless or out of control. The proposed wearable patient device and system connectable to the wearable patient device open new opportunities for receiving feedback from the patient as well as for providing feedback or guidance to the patient. Providing sensors in the wearable patient device for monitoring vital signs and other physiological parameters of the patient can improve the data available for examining the patient or refining the treatment. For example, in examinations requiring patients to respond to stimuli, the wearable patient device may provide additional feedback options that can easily be timed and synchronized to the stimuli. Using unique identifiers in components and messages and temporarily associating the components for an examination or treatment opens options for preparing patients while other patients are being examined or treated, thereby improving the workflow.

### BRIEF DESCRIPTION OF THE DRAWINGS

In the following section embodiments of the invention will be described, by way of example only, and with reference to the drawings in which:
Fig. 1 shows a first example of a wearable patient device in accordance with the present disclosure;
Fig. 2 shows a second example of a wearable patient device in accordance with the present disclosure;
Fig. 3 illustrates a first example of a system including a wearable patient device in accordance with the present disclosure;
Fig. 4 illustrates a second example of a system including a wearable patient device in accordance with the present disclosure;
Fig. 5 illustrates a third example of a system including a wearable patient device in accordance with the present disclosure;
Fig. 6 shows a first schematic message flow in a system according to figure 3;
Fig. 7 shows a second schematic message flow in a system according to figure 5; and
Fig. 8 shows a third schematic message flow in a system according to figure 5.

### DETAILED DESCRIPTION OF EMBODIMENTS

Like numbered elements in these figures are either identical or equivalent elements or perform the same function. Elements which have been discussed previously will not necessarily be discussed in later figures if the function is identical or equivalent.

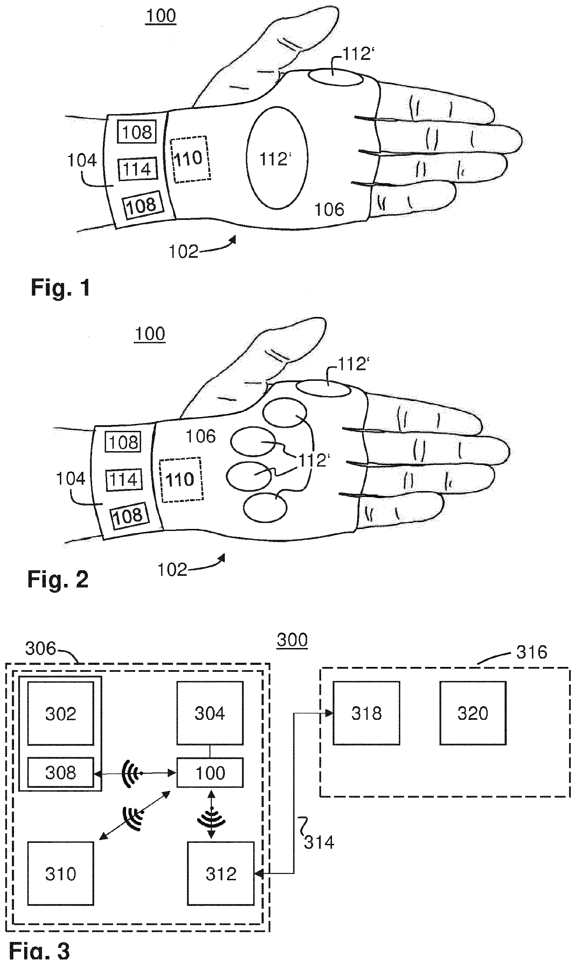

Fig. 1 shows a first example of a wearable patient device 100 in accordance with the present disclosure. The wearable patient device may comprise a glove-like enclosure 102 comprising a wrist section 104 and a palm section 106. Wrist section 104 includes e.g. various sensors 108 that are configured to measure physiological properties of the patient including, e.g., pulse rate, blood oxygenation, temperature, skin conductivity. Palm section 106 may include a communication module 110 that is configured to transmit communication and/or control signals to at least one receiver and to receive communication and/or control signals from at least one transmitter (both not shown in the figure). Communication module 110 may be placed on the back of the hand, as indicated by the dashed line. A power supply (not shown in the figure) may be provided with the wearable patient device. The enclosure may have flexible portions permitting attachment to the hand like a glove, or may be configured to be opened for easier attachment to a patient's hand. A closing mechanism (not shown in the figure) may be provided, e.g. two parts of a Velcro strip engaging with each other, and providing some flexibility to adapt the fit to hands of various sizes. Other closing mechanisms are likewise conceivable, including adjustable strap and buckle and the like. The closing mechanism may be located at the back of the hand. Communication module 110 may include or be connected to a microprocessor, memory (not shown in the figure) and input means 112 and/or output means 114 of a patient interface. Input means 112 may include switches or touch buttons 112' that are arranged on the enclosure 102 in such a way that they can be reached by moving the fingers of the hand, e.g. between the thumb and the first joint of the index finger and in the palm of the hand. Output means 114 may include a vibrator that produces perceivable vibrations, e.g. at the wrist. The memory included in the or connected to communication module 110 may be any combination of memory which is accessible to the microprocessor. This may include such things as main memory, cached memory, and also non-volatile memory such as flash RAM. In some examples the memory may be considered to be a non-transitory computer-readable medium. The memory may contain machine-executable instructions which enable the microprocessor to both control the operation and function of the wearable patient device.

Fig, 2 shows a second example of a wearable patient device in accordance with the present disclosure. The wearable patient device shown in this figure is very similar to the one discussed with reference to figure 1, but in this example a larger number of switches or touch buttons 112' of the input means 112 is provided, allowing for providing more patient feedback or control options.

Other variants and embodiments of wearable patient devices that are not shown in the figures may include larger enclosures 102 having an at least partially hard outer shell, or a number of flexible bracelet-like enclosures linked to each other that may be attached at appropriate positions of a lower part of a patient's arm (not shown in the figures). In another example, the wearable patient device may be attachable or wearable on a thigh area or an abdominal area or a hip area. The device could also be carried on the hand of the patient in such a way that any elements of the patient interface are located in the palm of the hand, so that the fingers associated with this palm could be used to operate the elements.

Fig. 3 illustrates a first example of a system 300 including a wearable patient device 100 in accordance with the present disclosure. A medical examination or treatment apparatus 302 for examination or treatment of a patient 304 is located in an examination or treatment room 306. The room may be shielded, the shielding indicated by the double dashed line. Medical examination or treatment device 302 may include a part 308 that is patient-controllable, e.g., a patient support having patient-adjustable cushions or the like. Examination or treatment room 306 may be considered a system associated with medical examination or treatment device 320 and may have one or more properties, environmental or other, that are patient-controllable and that can be controlled through room property controller 310. A receiver and/or transmitter 312 is provided inside examination or treatment room 306, which is connected to the outside of examination or treatment room 306 by connection 314, as will be discussed further below. Wearable patient device 100, which is attached to patient 304, as indicated by the line connecting the boxes, is configured to establish wireless connections with each of the patient-controllable part 308 of medical examination or treatment device 302, room property controller 310 and receiver and/or transmitter 312. The wireless connections may be unidirectional or bidirectional, as deemed appropriate for an actual system.

As mentioned above, receiver and/or transmitter 312 is connected to the outside of examination or treatment room 306, e.g. to control room 316 and, more particularly, to a communication system 318 configured to provide a communication connection between patient 304 and an operator 318 of the medical examination or treatment device 302, by means of connection 314. Connection 314 may be a physical point-to-point connection or a network providing one or more logical connections. For the sake of simplicity, control connections between control room 314, medical examination or treatment device 302 and room property controller 310 are omitted in this and the following figures.

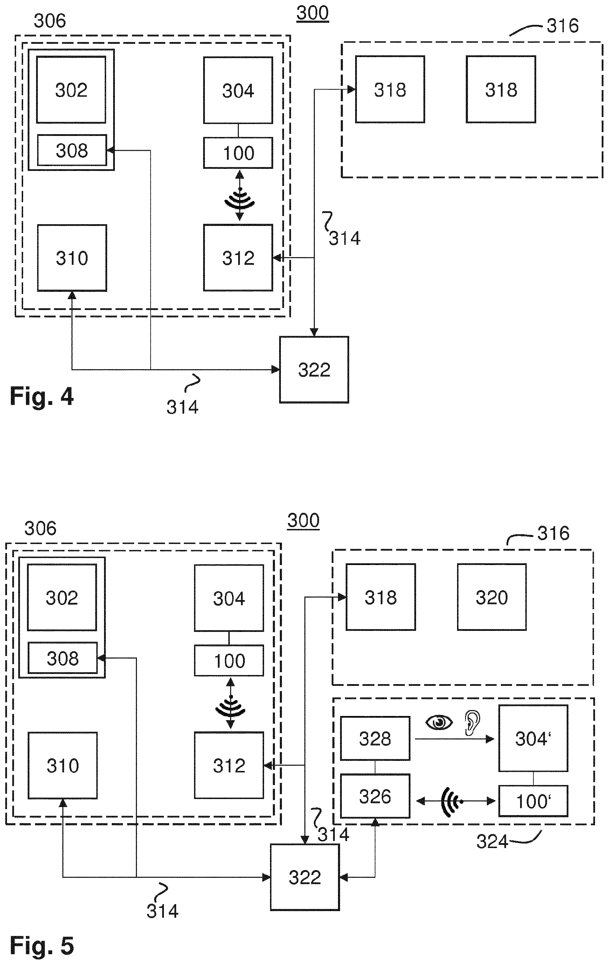

Fig. 4 illustrates a second example of a system 300 including a wearable patient device 100 in accordance with the present disclosure. The elements or components in examination or treatment room 306 are the same as presented with reference to figure 3. However, in this example medical examination or treatment apparatus 302 and the patient-controllable part 308 and room property controller 310 are not configured to establish a direct communication connection with wearable patient device 100. Rather, these elements are connected to system controller 322, which is itself connected to receiver and/or transmitter 312 and control room 316 or communication system 318, respectively. System controller 322 may reside outside examination or treatment room 306, as shown in the figure, or inside examination or treatment room 306 (not shown in the figures). Wearable patient device 100 is configured to establish a single wireless connection with receiver and/or transmitter 312. Any communication or signaling from or to wearable patient device 100 will be established and/or routed by system controller 322 via connection 314, which may comprise a number of physical point-to-point connections or a network that is configured to provide an appropriate number of logical connections.

Fig. 5 illustrates a third example of a system 300 including a wearable patient device 100 in accordance with the present disclosure. System 300 largely corresponds to the system presented with reference to figure 4, but additionally has a preparation or training room 324. Preparation or training room 324 includes training system comprising a computer 326 that is connected to a visual and/or acoustic interface 328. A further wearable patient device 100' is wirelessly connected to a corresponding interface of computer 326. Computer 326 executes program instructions representing a training program for the next patient 304', to which the further wearable patient device 100' is attached. The training program may include audio and/or video signals, or stimuli, issued to next patient 304' who, in response thereto, provides input to the patient interface of further wearable patient device 100'. The audio and/or video signals may represent instructions that may occur during an impending examination or treatment. Further wearable patient device 100' transmits the user input to computer 326 which compares the input with one or more possible or appropriate patient inputs for a given stimulus.

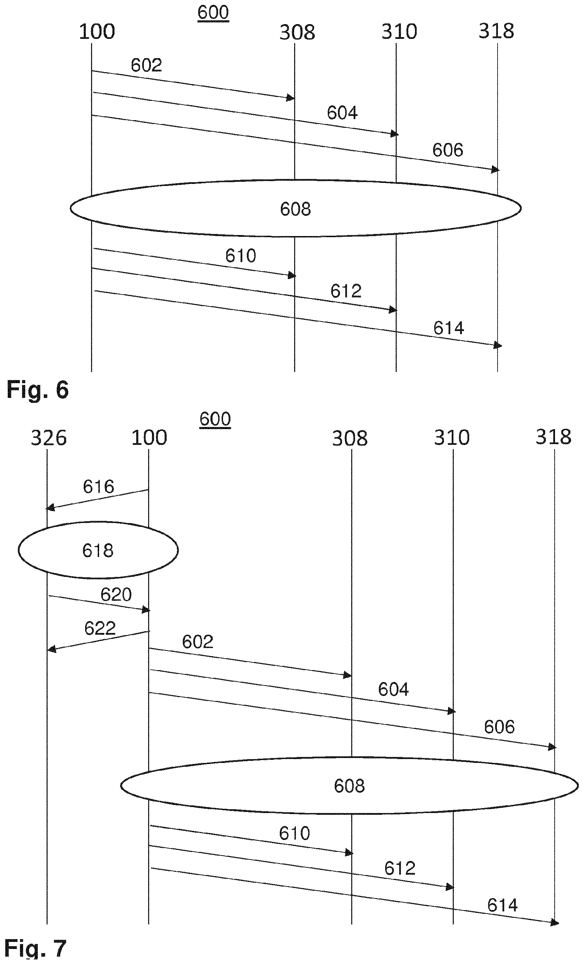

Fig. 6 shows a first exemplary schematic message flow 600 in a system according to figure 3, in which system the different connections may be managed through wearable patient device 100. Accordingly, wearable patient device 100 initiates a first connection with patient-controllable part 308 of medical examination or treatment apparatus 302 in step or message 602, a second connection with room property controller 310 in step or message 604, and a third connection with communication system 318 in step or message 606. It is to be noted that only single messages for initiating or terminating a communication are shown in this figure and the following figures for simplicity. Initiation and termination of communication connections may comprise sequences of messages going in both directions, including acknowledge (ACK) messages, which are likewise not shown in the figures. It is further to be noted that the sequence in which the connections are initiated may differ, and that connections may be initiated and terminated at any time during the examination or treatment. Initiation of a connection may be only preliminary or for testing general functions, and such connection may be activated only as required. In phase 608 communication between the wearable patient device and various elements of the system occurs as required during examination or treatment of the patient. At least after the examination or treatment has ended the wearable patient device terminates any of the first through third connections that may still be active by issuing corresponding termination messages 610, 612 and 614.

Fig. 7 shows a second exemplary schematic message flow in a system according to figure 4, in which a training for instructing the patient how to use the system precedes the actual examination or treatment. It is to be noted that the training or preparation room 324 and the elements thereof are not shown in figure 4, and reference is made to figure 5 for the respective elements. Wearable patient device 100 initiates a communication connection to training computer 326, and messages can be sent to and received by wearable patient device 100 and the training computer 326, respectively, in phase 618 as required by the training program running on training computer 326 until the training phase has ended. The training messages may include providing audio and visual stimuli or feedback to the patient through the visual and/or acoustic interface 328 connected to training computer 326. Once the training phase has ended the training computer informs wearable patient device accordingly by sending an according message 620. If message 620 does not also terminate the connection, wearable patient device may send an according termination message 622. Assuming that the and the treatment or examination room are now ready for treatment, wearable patient device initiates the communication connections with the various elements and components in the same way as has been described with reference to figure 6. The message that indicates a completed training may be necessary for enabling wearable patient device to establish connections with elements or components in the examination or treatment room 306, thereby preventing starting a treatment without proper training of the patient. An according software component within wearable patient device 100 may ensure that wearable patient device 100 can only initiate connections with patient-controllable part 308 of medical examination or treatment device 302, room property controller 310 and communication system 318 after computer 326 indicates a successfully completed training.

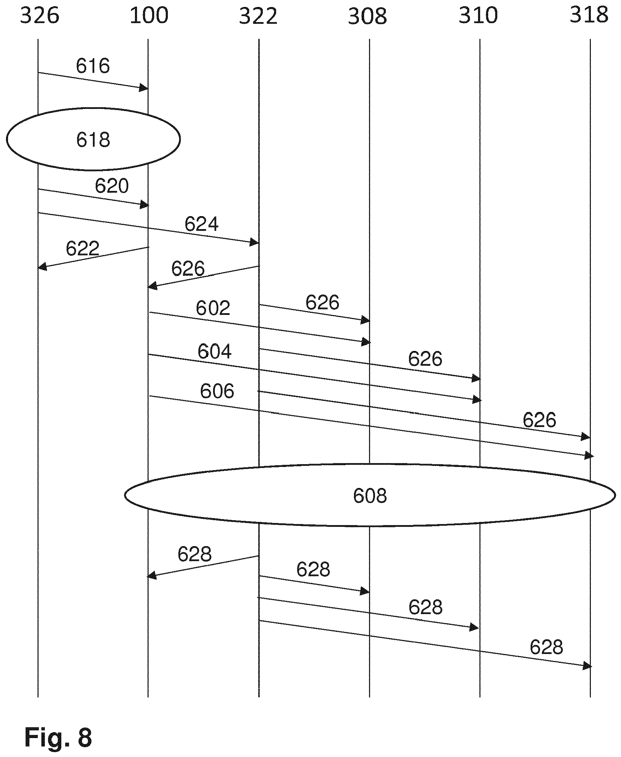

Fig. 8 shows a third exemplary schematic message flow in a system according to figure 5, in which at least some part of the communication between the wearable patient device and the elements and devices in the examination or treatment room and/or the training room is routed via central system controller 322. Initially, a patient must perform a training in training or preparation room 324, and the wearable patient device initiates the connection, performs the training phase and terminates the connection in the same way as has been described with reference to figure 7. In addition to sending the message indicating the completed training only to wearable patient device 100 training computer 326 sends a further message 624 indicating a completed training to central system controller 322, which may, assuming that the patient and the treatment or examination room are now ready for treatment, send configuration messages 626 to wearable patient device 100, patient-controllable part 308 of medical examination or treatment device 302, room property controller 310 and communication system 318 for sharing according connection information and/or authorization and/or authentication information. Wearable patient device may connect to the aforementioned elements in the same way as has been described with reference to figure 6, using the connection information and/or authorization and/or authentication information provided by system controller 322. Wearable patient device may then communicate with any of the elements in the system during the examination or treatment phase 608 in the same way as has been described further above. Once the treatment or examination has ended system controller may send termination messages 628 to wearable patient device 100, patient-controllable part 308 of medical examination or treatment device 302, room property controller 310 and communication system 318. Termination message 628 may include de-authorization and/or de-authentication to the various elements, for preventing inadvertent reestablishing a communication.

It is to be noted that the systems discussed hereinbefore show only exemplary setups, and that mixed configurations are also conceivable. Accordingly, some of the message flows that have been presented in relation to the exemplary systems may be different. In particular, communication connections may be initiated and/or terminated by wearable patient device or central system controller 322, if such system controller is present in a system, but messages may be sent directly from wearable patient device 100 to the connected elements.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: wearable patient device
- 100': further wearable patient device
- 102: enclosure
- 104: wrist section
- 106: palm section
- 108: sensor
- 110: communication module
- 112: input means
- 112': switch/button
- 114: output means
- 300: system
- 302: medical apparatus
- 304: patient
- 304': next patient
- 306: examination or treatment room
- 308: patient-controllable part
- 310: room property controller
- 312: receiver and/or transmitter
- 314: connection
- 316: control room
- 318: communication system
- 320: operator
- 322: system controller
- 324: preparation/training room
- 326: system simulator
- 328: visual/acoustic interface
- 600: message flow
- 602: initiation message
- 604: initiation message
- 606: initiation message
- 608: examination/treatment phase comm.
- 610: termination message
- 612: termination message
- 614: termination message
- 616: initiation message
- 618: training phase communication
- 620: "training ended" message
- 622: termination message
- 624: "training ended" message
- 626: connection information message
- 628: termination message

## Claims

1. A medical system (300) comprising a medical examination or treatment apparatus (302) and a wearable patient device (100), the medical examination or treatment apparatus (302) comprising an examination or treatment zone for a patient (304), the wearable patient device (100) comprising a user interface operable by a hand of the patient when the patient (304) is positioned in the examination or treatment zone of the medical examination or treatment apparatus (302), wherein the wearable patient device (100) is communicatively connected with the medical examination or treatment apparatus (302) via a wireless connection, for sending a control command corresponding to input received from the patient via the user interface of the wearable patient device (100), the control command being adapted to control a patient-controllable part or parameter (308) of the medical examination or treatment apparatus (302).

2. The medical system (300) of claim 1, wherein the controlling of the patient-controllable part comprises one or more of interrupting or aborting an examination or treatment, controlling at least a temperature or a softness of a cushion of a patient bed or patient support for positioning the patient (304) in the examination or treatment zone, a system for illuminating, ventilating or odorizing of the examination or treatment zone.

3. The medical system (300) of claim 1, wherein the wearable patient device (100) further comprises one or more sensors (108) configured to detect physiological properties or parameters of the patient (304), wherein the wearable patient device (100) is adapted for transmitting via the wireless connection a signal representing the sensor input, or derivatives thereof, to the medical examination or treatment apparatus (302), and wherein the medical examination or treatment apparatus (302) is adapted to modify its operation in response to the received signal.

4. The medical system (300) of claim 3, wherein the sensors (108) of the wearable patient device (100) comprise sensors for determining as the physiological properties or parameters a patient's heart rate or pulse, blood pressure, blood oxygenation, blood glucose level, skin conductivity, temperature, gyroscopes or accelerometers.

5. The medical system (300) of claim 3 or 4, wherein modifying the operation of the medical examination or treatment apparatus (302) comprises one or more of interrupting or aborting an examination or treatment of the patient by the medical examination or treatment apparatus (302), or triggering an examination or treatment or a particular variant thereof of the patient by the medical examination or treatment apparatus (302).

6. The medical system (300) of claim 1, wherein the wearable patient device (100) is further communicatively connected with a communication system (318) providing voice communication between the patient and an operator of the medical examination or treatment device (302), and wherein the wearable patient device (100) is configured to establish or initiate the voice communication.

7. The medical system (300) of claim 1, wherein the medical examination or treatment apparatus (320) is configured to selectively communicatively connect with individual ones of a plurality of wearable patient devices (100).

8. The medical system (300) of claim 1, wherein the examination or treatment apparatus (302) and/or the wearable patient device (100) are configured to periodically or continuously monitor the communication connection therebetween.

9. The medical system (300) of claim 1, wherein the examination or treatment apparatus (302) is configured to trigger provision of feedback to the patient through corresponding output means of the wearable patient device (100).

10. The medical system (300) of claim 1, wherein the medical diagnostic or treatment apparatus (302) is a diagnostic imaging device, including a PET system, a CT system, an MRI system, a SPECT system, an X-Ray system, a gamma ray treatment system, or a high intensity ultrasound system.

11. A method of controlling a medical examination or treatment apparatus (320) by a wearable patient device (100), the medical examination or treatment apparatus (320) comprising an examination or treatment zone for a patient (304), the wearable patient device (100) comprising a user interface operable by a hand of the patient when the patient (304) is positioned in the examination or treatment zone of the medical examination or treatment apparatus (302), wherein the wearable patient device (100) is communicatively connected with the medical examination or treatment apparatus (302) via a wireless connection, the method comprising:
- sending by the wearable patient device (100) a control command corresponding to input received from the patient via the user interface of the wearable patient device (100),
- in response to receiving the control command controlling by the medical examination or treatment apparatus (320) patient-controllable parts or parameters (308) of the medical examination or treatment apparatus (302).

12. The method of claim 11, further comprising
temporarily associating and communicatively connecting the wearable patient device (100) and the medical examination or treatment apparatus (302).

13. The method of claim 12, further comprising testing the function of the wearable patient device (100) and its operability by the patient (304) prior to the associating and connecting of the wearable patient device (100) and the medical examination or treatment apparatus (302).

14. A computer program product comprising computer executable instructions for performing the method of any of the previous claims 11-13.
